Europäisches Patentamt

European Patent Office

Office européen des brevets

(19)

(11) Numéro de publication: **0 188 979**
**B1**

(12) **FASCICULE DE BREVET EUROPEEN**

(45) Date de publication du fascicule du brevet:
**09.03.88**

(51) Int. Cl.⁴: **C 07 C 46/00,** C 07 C 50/18,
D 21 C 3/02

(21) Numéro de dépôt: **85420233.0**

(22) Date de dépôt: **19.12.85**

(54) **Procédé de préparation d'anthraquinone.**

(30) Priorité: **21.12.84 FR 8420090**

(43) Date de publication de la demande:
**30.07.86 Bulletin 86/31**

(45) Mention de la délivrance du brevet:
**09.03.88 Bulletin 88/10**

(84) Etats contractants désignés:
**AT BE CH DE FR GB IT LI NL SE**

(56) Documents cités:
**FR - A - 2 279 710**
**US - A - 1 591 712**
**US - A - 3 089 879**

**Le dossier contient des informations techniques
présentées postérieurement au dépôt de la demande et
ne figurant pas dans le présent fascicule.**

(73) Titulaire: **ATOCHEM, 4 & 8, Cours Michelet La
Défense 10, F-92800 Puteaux (FR)**

(72) Inventeur: **Devic, Michel, 27 Chemin des Fonds,
F-69110 Ste Foy Les Lyon (FR)**
Inventeur: **Schirmann, Jean-Pierre, 49 chemin de la
Glacière, F-69600 Oullins (FR)**

ACTORUM AG

## Description

La présente invention concerne un procédé de préparation d'anthraquinone.

Elle concerne plus particulièrement un procédé modifié d'obtention de l'anthraquinone par cyclisation de l'acide orthobenzoylbenzoïque en présence d'acide sulfurique.

Dans l'industrie, en plus de son emploi dans le domaine des matières colorantes, l'anthraquinone est de plus en plus utilisée en particulier dans l'industrie papetière pour améliorer la cuisson alcaline du bois comme cela est par exemple décrit dans le brevet français publié sous le N° 2 373 637.

Le rôle de catalyseur de délignification que joue alors l'anthraquinone est constaté à ce jour lorsque ce produit est mis en œuvre sous une forme hautement purifiée qui demande donc à être préalablement atteinte.

Malheureusement, une telle présentation de l'anthraquinone pèse évidemment sur l'économie d'une industrie comme l'industrie papetière qui aboutit à un produit de faible valeur marchande.

Le problème technique que la titulaire s'est attachée à résoudre a été de définir un procédé conduisant à de l'anthraquinone dont l'application dans une industrie telle que l'industrie papetière garantisse un gain économique sans perte d'efficacité.

Il est connu de cycliser l'acide orthobenzoylbenzoïque, désigné dans ce qui suit acide O.B.B., par chauffage en présence d'une quantité importante d'acide sulfurique concentré ou d'oléum (voir par exemple I. VOGEL, «Practical Organic Chemistry», 3e édition, p. 740), d'isoler l'anthraquinone ainsi formée et de la purifier, par exemple en provoquant sa précipitation par dilution du milieu de réaction à l'aide d'un grand volume d'eau en isolant par filtration l'anthraquinone précipitée et en la lavant ensuite abondamment à l'eau jusqu'à neutralité pour en éliminer au maximum les impuretés résiduelles avant de procéder à son séchage. L'anthraquinone ainsi purifiée est généralement conditionnée avant emploi dans l'industrie papetière, sous la forme d'une dispersion dans un produit solide inerte, renfermant 50% en poids d'anthraquinone et obtenu par broyage fin des deux constituants en présence d'un agent dispersant.

Les modes de cyclisation de l'acide O.B.B. en vue d'obtenir de l'anthraquinone purifiée telle qu'elle est à ce jour employée dans l'industrie entraînent non seulement un coût direct élevé de ce produit mais encore un coût additionnel conséquent dû à la dépollution des effluents acides avant rejet. Cette valeur initiale élevée de l'anthraquinone est évidemment peu compatible avec son emploi dans une industrie comme l'industrie papetière qui exige des matières premières de coût réduit.

Des tentatives ont été faites pour rendre plus économique l'obtention de l'anthraquinone à partir d'acide O.B.B.

Elles ont consisté par exemple soit à diminuer la quantité d'acide sulfurique comme cela est décrit dans la demande de brevet japonais N° 49-7260/74 et le brevet des Etats-Unis d'Amérique N° 2 842 562, soit à substituer à l'acide sulfurique un autre catalyseur, comme l'argile proposée dans le certificat d'utilité français N° 76.18956, le brevet japonais N° 49-6240/74 ou la demande de brevet français publiée sous le N° 2 545 483.

Dans tous les cas ci-dessus, les procédés visant à l'obtention d'anthraquinone purifiée, les avantages dégagés au niveau d'un moindre coût possible des matières sont économiquement contrecarrés par le coût plus élevé qu'entraîne une plus grande complexité de l'équipement nécessaire.

Le procédé de l'invention conduit à l'anthraquinone sous une forme qui autorise sa mise en œuvre à la fois économique et efficace dans l'industrie papetière.

Il est caractérisé en ce que le mélange de réaction résultant de la cyclisation de l'acide O.B.B. à une température comprise entre 150 et 180°C et une pression absolue comprise entre 5 et 40 millibars, en présence de 0,5 à 2 parties en poids d'acide sulfurique titrant au moins 95% en poids par partie d'acide O.B.B., est directement amené à un pH égal au minimum à 7 par addition, à une température comprise entre la température ambiante et 100°C, d'un hydroxyde d'un métal alcalin choisi parmi l'hydroxyde de sodium et l'hydroxyde de potassium sous la forme d'une solution aqueuse renfermant de 10 à 40% en poids d'hydroxyde, avant de réaliser, par des moyens connus en soi, la dispersion de l'anthraquinone formée au sein de la suspension ainsi obtenue ou de ses constituants amenés à l'état sec à partir de ladite dispersion.

Dans les gammes de valeurs exposées ci-dessus, qui conviennent particulièrement bien à la réalisation du procédé selon l'invention, celles qui sont optimales sont les suivantes:

— lors de la cyclisation de l'acide O.B.B.:

quantité relative d'acide sulfurique et d'acide O.B.B.: 0,6 à 1 partie en poids de $H_2SO_4$ par partie d'acide O.B.B.,

température, pression absolue: environ 160°C, 10 millibars,

— lors de l'addition de l'hydroxyde de métal alcalin:

température, pression: 50 à 60°C, pression préférée égale à la pression atmosphérique pour des raisons de commodité d'exécution mais pouvant être inférieure ou supérieure à cette valeur,

concentration de la solution d'hydroxyde: 20 à 30% en poids.

Dans les conditions générales de réalisation du procédé, la durée de l'opération est généralement comprise entre 30 minutes et 3 heures. Dans les conditions préférentielles énoncées, cette durée est de l'ordre de 1 heure.

La dispersion de l'anthraquinone au sein de la suspension résultant de l'addition de la solution d'hydroxyde peut être réalisée par tout moyen efficace connu, par exemple au moyen d'un broyeur à disque tournant avec du sable en présence, préférentiellement, d'un agent dispersant tel que par exemple le lignosulfonate de sodium.

La dispersion ainsi obtenue est directement applicable dans l'industrie papetière.

La dispersion à l'état sec de l'anthraquinone à partir de la dispersion à l'état humide ci-dessus résulte du séchage de celle-ci par tout moyen approprié connu, par exemple l'atomisation dans un courant d'air chaud, après apport des additifs traditionnels en pareil cas tels qu'un antimottant et un antipoussière.

Le procédé de l'invention est particulièrement économique puisqu'il ne comporte plus en particulier les étapes de filtration, de lavage, de traitement d'effluents pollués par des matières acides et/ou organiques.

Par contre, il devait être normalement attendu que la présence de telles impuretés comme, entre autres, l'acide O.B.B. non transformé, l'acide phtalique, les produits polycondensés, à côté de l'anthraquinone, perturbe fortement l'activité catalytique spécifique de ce produit au cours de la délignification du bois.

Il ressort des exemples suivants, donnés à titre indicatif et non limitatif, qu'il n'en est rien et que l'invention permet de concilier avantageusement et d'une façon inattendue économie et efficacité.

*Exemple 1 :*

*Préparation de la dispersion d'anthraquinone selon l'invention*

Dans un évaporateur rotatif en verre sont chauffés durant 1 heure à 160°C et à une pression absolue de 10 millibars 22,6 g d'acide O.B.B. pur commercialisé par la Société MERCK et 22,6 g de $H_2SO_4$ à 96% en poids.

Cette opération terminée, le mélange est mis à pression atmosphérique et, en maintenant la température à 80°C, son pH est porté à 9 par addition sous agitation d'une solution d'hydroxyde de sodium renfermant 30% en poids de NaOH.

La suspension résultante est broyée dans un mortier pour former une dispersion dont le titre en anthraquinone est ajusté à 20% en poids par addition d'eau.

*Application de la dispersion dans la délignification du bois*

Des copeaux de pin maritime de la forêt landaise sont traités dans un bain alcalin de cuisson dont le taux d'alcalinité (hydroxyde de sodium) est de 23%, le rapport pondéral liqueur/végétal égal à 4, selon le programme suivant: durée de montée à la température de 170°C: 1,5 heure, durée du palier de température à 170°C: 1,5 heure.

L'indice Kappa (norme française AFNOR N.F. T 12 018) de la pâte papetière finalement obtenue est déterminé lorsque l'essai est effectué dans les conditions ci-dessus (essai I), dans les mêmes conditions mais en présence d'une quantité variable soit d'anthraquinone purifiée titrant 99% telle que livrée par la Société BAYER A.G. (essai II) soit d'anthraquinone contenue dans la dispersion à 20% de l'exemple 1 (essai III).

La quantité d'anthraquinone engagée est chaque fois exprimée en pourcent en poids du poids de bois sec.

Le tableau I rassemble les résultats obtenus exprimés par les indices Kappa des pâtes obtenues, qui illustrent l'efficacité de la délignification.

*Tableau I*

| Essai | Anthraquinone % | Indice Kappa |
|-------|-----------------|--------------|
| I | 0 | 78,5 |
| II | 0,05 | 46,8 |
| | 0,20 | 31,0 |
| | 0,80 | 22,0 |
| III | 0,05 | 45,6 |
| | 0,20 | 32,3 |
| | 0,80 | 29,8 |

*Exemple 2 :*

En procédant comme dans l'exemple 1 mais en ajoutant la solution d'hydroxyde de sodium à une température de 50°C pour amener le pH du milieu de réaction à 9, en procédant à un séchage à l'étuve à 110°C de la dispersion obtenue après la phase de broyage au mortier, en effectuant un broyage à nouveau après séchage, on obtient finalement 50,8 g d'une poudre renfermant 40% en poids d'anthraquinone.

Cette poudre est employée en lieu et place de la dispersion de l'exemple 1 dans un essai en tout point identique par ailleurs à l'essai III du tableau I.

Pour 0,05%, 0,20% et 0,80% d'anthraquinone sont respectivement obtenus les indices Kappa suivants: 46,3, 33,4 et 22,8.

*Exemple 3 :*

En procédant selon l'exemple 2 avec 22,6 g d'acide O.B.B. qualité MERCK et 14,95 g d'acide sulfurique titrant 96% en poids, une pression absolue de 6 millibars durant l'heure de cyclisation à 160°C, une addition d'hydroxyde de sodium telle que le pH du mélange avant broyage soit de 9, on obtient finalement 41 g d'une poudre renfermant 50% en poids d'anthraquinone à l'état dispersé.

Mise en œuvre dans des tests de cuisson alcaline du bois tels que ceux déjà décrits sous la désignation essai III, il est constaté, pour l'anthraquinone contenue, une efficacité semblable pratiquement en tout point à celle de l'anthraquinone purifiée.

**Revendications**

1. Procédé modifié d'obtention d'anthraquinone par cyclisation de l'acide orthobenzoylbenzoïque, caractérisé en ce que le mélange de réaction résultant de la cyclisation de l'acide orthobenzoylbenzoïque à une température comprise entre 150 et 180°C et une pression absolue comprise entre 5 et 40 millibars en présence de 0,5 à 2 parties en poids d'acide sulfurique titrant au moins 95% en poids, par partie d'acide orthobenzoylbenzoïque, est directement amené à un pH égal au minimum à 7 par addition, à une température comprise entre la température ambiante et 100°C, d'un hydroxyde d'un métal alcalin choisi parmi l'hydroxyde de sodium et l'hydroxyde de potassium sous la forme d'une solution aqueuse renfermant de 10 à 40% en poids d'hydroxyde, avant de réaliser, par des moyens connus en soi, la dispersion de l'anthraquinone formée au sein de la suspension ainsi obtenue ou de ses constituants amenés à l'état sec à partir de ladite dispersion.

2. Procédé selon la revendication 1, caractérisé en ce que l'addition de l'hydroxyde de métal alcalin est réalisée à une température comprise entre 50 et 60°C.

3. Procédé selon l'une des revendications 1 et 2, caractérisé en ce que la solution d'hydroxyde de métal alcalin renferme de 20 à 40% en poids d'hydroxyde.

4. Application dans l'industrie papetière de la dispersion à l'état humide obtenue selon la revendication 1 comme additif aux bains alcalins de cuisson des bois.

5. Application dans l'industrie papetière de la dispersion à l'état sec obtenue selon la revendication 1 comme additif aux bains alcalins de cuisson des bois.

## Patentansprüche

1. Modifiziertes Verfahren zur Herstellung von Anthrachinon durch Zyklisierung von Orthobenzoylbenzoesäure, dadurch gekennzeichnet, dass das bei der Zyklisierung von Orthobenzoylbenzoesäure bei einer Temperatur zwischen 150 und 180°C und einem absoluten Druck zwischen 5 und 40 Millibar in Gegenwart von 0,5 bis 2 Gewichtsteilen einer wenigstens 95 gewichtsprozentigen Schwefelsäure pro Teil Orthobenzoylbenzoesäure erhaltene Reaktionsgemisch direkt bei einer Temperatur zwischen der Umgebungstemperatur und 100°C durch Zugabe eines Hydroxids eines Alkalimetalls, ausgewählt aus Natriumhydroxid und Kaliumhydroxid, in Form einer wässerigen Lösung, die wenigstens 10 bis 40 Gew.-% Hydroxid enthält, auf einen pH-Wert von wenigstens 7 gebracht wird, bevor in an sich bekannter Weise das in der so erhaltenen Suspension gebildete Anthrachinon dispergiert wird oder ausgehend von dieser Dispersion, die in den trockenen Zustand übergeführten Bestandteile der Suspension fein verteilt werden.

2. Verfahren nach Anspruch 1, dadurch gekennzeichnet, dass die Zugabe des Alkalimetallhydroxids bei einer Temperatur zwischen 50 und 60°C durchgeführt wird.

3. Verfahren nach einem der Ansprüche 1 und 2, dadurch gekennzeichnet, dass die Lösung des Alkalimetallhydroxids 20 bis 30 Gew-% an Hydroxid enthält.

4. Verwendung der gemäss Anspruch 1 erhaltenen Dispersion in feuchtem Zustand in der Papierindustrie als Additiv für alkalische Holzkochlaugen.

5. Verwendung der gemäss Anspruch 1 erhaltenen Dispersion in trockenem Zustand in der Papierindustrie als Additiv für alkalische Holzkochlaugen.

## Claims

1. Modified process for producing anthraquinone by cyclization of ortho-benzoylbenzoic acid, characterized in that the reaction mixture resulting from the cyclization of ortho-benzoylbenzoic acid at a temperature between 150 und 180°C and an absolute pressure between 5 and 40 millibars in the presence of 0.5 to 2 parts by weight of sulphuric acid whose strength is determined as at least 95% by weight, per part of ortho-benzoylbenzoic acid, is directly brought to a pH of not less than 7 by the addition, at a temperature between ambient temperature and 100°C, of an alkali metal hydroxide chosen from sodium hydroxide and potassium hydroxide in the form of an aqueous solution containing from 10 to 40% by weight of hydroxide, before producing, by means known per se, the dispersion of the anthraquinone formed within the suspension produced in this manner or of its constituents produced in the dry state from the said dispersion.

2. Process according to Claim 1, characterized in that the addition of the alkali metal hydroxide is performed at a temperature between 50 and 60°C.

3. Process according to either of Claims 1 and 2, characterized in that the solution of alkali metal hydroxide contains from 20 to 30% by weight of hydroxide.

4. Application in the papermaking industry of the dispersion obtained according to Claim 1, in the wet state, as an additive to the alkaline baths for cooking woods.

5. Application in the papermaking industry of the dispersion obtained according to Claim 1, in the dry state, as an additive to the alkaline baths for cooking woods.